# EUROPÄISCHE PATENTANMELDUNG

(11) **EP 1 216 979 A1**
(43) Veröffentlichungstag der Anmeldung: **26.06.2002**
(21) Anmeldenummer: 01128486.6
(22) Anmeldetag: 07.12.2001
(51) Int. Cl.: C07C 29/38, C07C 31/22, C07C 51/02, C07C 53/02

(54) **Verfahren zur Herstellung von Trimethylolverbindungen und Ameisensäure**

(30) Priorität: 20.12.2000 DE 10063937
(71) Anmelder: BAYER AG, 51368 Leverkusen (DE)
(72) Erfinder: Döbert, Frank, Dr., 50933 Köln (DE); Wagner, Paul, Dr., 40597 Düsseldorf (DE); Klausener, Alexander, Dr., 50259 Pulheim (DE); Eymann, Wolfgang, Dr., 51061 Köln (DE); Feller, Rolf, 41352 Korschenbroich (DE)

(57) **Zusammenfassung**

Die vorliegende Erfindung betrifft ein Verfahren zur Herstellung von Trimethylolverbindungen und Ameisensäure durch Umsetzung von Formaldehyd und Aldehyden in Gegenwart einer Stickstoffbase und Destillation des erhaltenen Reaktionsgemisches in Gegenwart eines Hilfsstoffes.

## Beschreibung

Die vorliegende Erfindung betrifft ein Verfahren zur Herstellung von Trimethylolverbindungen und Ameisensäure durch Umsetzung von Formaldehyd und Aldehyden in Gegenwart einer Stickstoffbase und Destillation des erhaltenen Reaktionsgemisches in Gegenwart eines Hilfsstoffes.

Trimethylolverbindungen finden vielfach Verwendung im Kunststoffsektor zur Herstellung von Lacken, Urethanen und Polyestem. Wichtige Trimethylolverbindungen sind beispielsweise Trimethylolethan oder Trimethylolbutan, vor allem aber Trimethylolpropan.

Die industrielle Herstellung von Trimethylolpropan (TMP) geht von n-Butyraldehyd und Formaldehyd aus, welche in einem zweistufigen Reaktionsprozess umgesetzt werden. In einer ersten Reaktionsstufe wird zunächst in einer basenkatalysierten Aldolkondensation über die Zwischenstufe 2-Methylolbutanal 2,2-Dimethylolbutanal gebildet. In einer nachfolgenden gekreuzten Cannizzaro-Reaktion erfolgt unter Einwirkung stöchiometrischer Mengen einer Base die Bildung von Trimethylolpropan unter gleichzeitiger Bildung von Formiatsalzen.

Als Base werden meist anorganische Verbindungen wie beispielsweise Natrium-oder Calciumhydroxid eingesetzt. Wird im Verfahren Calciumhydroxid als Base eingesetzt, so kann das als Koppelprodukt anfallende Calciumformiat beispielsweise zur Herstellung verschiedener Tierfutterprodukte und Tierfutterproduktzusätze weiter verwendet werden. Das bei Einsatz von Natriumhydroxid anfallende Natriumformiat ist allerdings weniger gewünscht. Der Anfall eines anorganischen Formiatsalzes als Koppelprodukt ist allerdings auch bei möglicher Verwertung mit Nachteilen verbunden: zum einen ist die Abtrennung des Salzes von TMP kompliziert und erfordert zusätzlichen Aufwand, zum anderen muss das Salz - falls es nutzbringend verwertet werden soll - aufgearbeitet und gereinigt werden.

In einer alternativen Verfahrensvariante wird die Umsetzung von n-Butyraldehyd mit Formaldehyd in Gegenwart eines tertiären Amins, meist eines Trialkylamins, durchgeführt. Die verwendeten Überschüsse an Formaldehyd und Trialkylamin haben jedoch zur Folge, dass neben TMP stöchiometrische Mengen an Trialkylammoniumformiat entstehen. Um die Wirtschaftlichkeit eines solchen Verfahrens zu erhöhen, ist es notwendig, das eingesetzte Amin aus Trialkylammoniumformiat wieder zu gewinnen, wobei vorzugsweise das Formiat einer sinnvollen Verwendung zugeführt wird.

In DE 25 07 461 A ist ein Verfahren zur Herstellung von 2,2-Dimethylolalkanalen beschrieben, welche in einer anschließenden Hydrierung zu der entsprechenden Trimethylolverbindung umgesetzt werden können. So wird beispielsweise TMP durch Umsetzung von n-Butyraldehyd mit Formaldehyd in Gegenwart katalytischer Mengen tertiärer Amine und anschließender Hydrierung des Reaktionsproduktes hergestellt. Nachteilig an diesem Verfahren ist, dass nur unbefriedigende Ausbeuten an Trimethyolpropan erzielt werden.

In DE 1 952 738 A wird ein Verfahren zur TMP-Herstellung durch Umsetzung von n-Butyraldehyd mit Formaldehyd in Gegenwart tertiärer Amine beschrieben. Die dabei entstehenden Formiatsalze werden destillativ von TMP abgetrennt. Es wird vorgeschlagen, die entstehenden Trialkylammoniumformiate mit einer wässrigen Calciumhydroxidlösung zu Calciumformiat umzusetzen und das freiwerdende Amin in den Reaktionskreislauf zurückzuführen. Nachteilig an diesem Verfahren ist, dass wiederum ein anorganisches Formiatsalz anfällt, welches, falls es einem weiteren Verwendungszweck zugeführt werden soll, in einem weiteren Reaktionsschritt abgetrennt und aufgereinigt werden muss. Zudem muss als zusätzlicher Ausgangsstoff Calciumhydroxid eingesetzt werden, um das Formiat in ein verwertbares Produkt zu überführen.

In EP 142 090 A wird zur Herstellung von TMP ein mol n-Butyraldehyd mit 2,2 bis 4,5 mol Formaldehyd und 0,6 bis 3 mol Trialkylamin zur Reaktion gebracht und das entstehende 2,2-Dimethylolbutanal katalytisch hydriert. Nachteilig ist, dass durch die hohen Aminkonzentrationen bei der Aldolreaktion erhebliche Mengen an Trialkylammoniumformiaten entstehen, welche vor der Hydrierung abdestilliert werden müssen. Eine Freisetzung von Trialkylamin aus den entstandenen Formiaten und Rückführung der Base in das Verfahren wird nicht beschrieben.

Bei der in DE 28 13 201 A beschriebenen Methode zur Reduzierung des Formiatanfalls wird Formaldehyd im Überschuss eingesetzt, das Amin jedoch nur als Katalysator für die Aldolreaktion zum 2,2-Dimethylolbutanal eingesetzt. Der gebildete Aldehyd wird anschließend katalytisch hydriert. Das Verfahren ist wirtschaftlich wenig geeignet, da der Formaldehydüberschuss vor der Hydrierung wegen einer möglichen Vergiftung des Hydrierkatalysators abgetrennt werden muss.

In Anlehnung an EP 142 090 A wird in EP 289 921 A ein Herstellverfahren für Trimethylolalkane beschrieben, wobei 1 mol Aldehyd mit 2,2 bis 4,5 mol Formaldehyd in wässriger Lösung in Gegenwart von 0,6 bis 3 mol Trialkylamin umgesetzt und anschließend hydriert wird. Zur Aufarbeitung des anfallenden Trialkylammoniumformiats werden zwei Verfahrensvarianten angeführt. Nach Variante a) wird das rohe Hydriergemisch auf 100 bis 200°C erhitzt, wobei Wasser und überschüssiges Trialkylamin destillativ abgetrennt werden. Das im Sumpf verbleibende Trialkylammoniumformiat reagiert mit dem vorliegenden Alkohol zu Trimethyolalkanformiat, wodurch das eingesetzte Amin freigesetzt wird. Trimethylolalkanformiat wird anschließend mit Methanol zur Methylformiat und Trimethylolalkan umgeestert. In der Verfahrensvariante b) wird zunächst das Hydrierprodukt weitestgehend entwässert und das im Sumpf verbleibende Trialkylammoniumformiat wird im Anschluss mit Methanol direkt zu Methylformiat verestert. Nachteilig an beiden Varianten ist, dass zum Erreichen von wirtschaftlichen Ausbeuten ein Verlust an Formaldehyd durch die katalytische Hydrierung in Kauf genommen werden muss.

In DE 195 42 036 A erfolgt die Rückführung des als Base eingesetzten tertiären Amins durch Veresterung des entstandenen Trialkylammoniumformiats mit Polymethylolalkan. Nachteilig an dieser Methode ist, dass der entstehende Ester in einem weiteren Schritt zur Freisetzung des Polymethylolalkans mit einem weiteren, leichter siedenden Alkohol umgeestert werden muss.

In WO 98/28253 A wird ein Verfahren zur Herstellung von TMP ohne den Anfall eines Koppelprodukts beschrieben. Dabei wird in einer ersten Reaktionsstufe n-Butyraldehyd mit der 2- bis 8-fachen molaren Menge Formaldehyd in Gegenwart eines tertiären Amins als Katalysator umgesetzt. Das dabei entstehende Reaktionsgemisch wird in einer zweiten Stufe destillativ aufgetrennt, wobei der aus überwiegend nicht umgesetzten oder teilumgesetzten Ausgangsmaterialien bestehende Destillatstrom in die erste Stufe zurückgeführt wird und der überwiegend 2,2-Dimethylolalkanal enthaltende Sumpf abgetrennt wird oder das Reaktions-gemisch aus der ersten Stufe durch Phasentrennung in eine wässrige und eine organische Phase getrennt wird und die organische Phase in die erste Stufe zurückgeführt wird. In einer dritten Nachreaktionsstufe wird die abgetrennte Sumpffraktion der zweiten Stufe oder die in der zweiten Stufe durch Phasentrennung erhaltene wässrige Phase einer katalytischen und/oder thermischen Behandlung unterworfen, wobei die unvollständig umgesetzten Verbindungen in 2,2-Dimethyolalkanal und Ausgangsprodukte, die in die erste Stufe zurückgeführt werden, überführt werden. Anschließend wird in an sich bekannter Weise 2,2-Dimethylolalkanal zur entsprechenden Trimethylolverbindung hydriert. Nachteilig an diesem Verfahren ist allerdings, dass das entstehende Monomethyolalkanal durch aufwendige Maßnahmen aus dem Reaktionsgemisch eliminiert werden muss, da ansonsten bei der katalytischen Hydrierung größere Mengen an Nebenprodukten entstehen.

Ein weiteres Verfahren zur Rückgewinnung der eingesetzten Amine ist in DE 198 48 568 A und DE 198 48 569 A offenbart. Nach Umsetzung des Aldehyds mit wässrigem Formaldehyd in Gegenwart eines tertiären Amins wird das Reaktionsgemisch zunächst destillativ von freiem Amin und Wasser befreit. Das im Sumpf verbleibende Trialkylammoniumformiat wird durch Destillation bei einem pH-Wert von 5 soweit eingeengt, das sich Trimethylolalkanformiat und freies Amin bildet, welches als Destillat abgetrennt wird. Das Trimethylolalkanformiat wird katalytisch unter Druck bei Temperaturen um 280°C zu Trimethylolalkan, Wasserstoff, Kohlendioxid, Wasser und Kohlenmonoxid zersetzt. Nachteilig bei diesem Verfahren ist, dass mindestens 1 Moläquivalent Formaldehyd pro mol Alkanal wirtschaftlich nicht genutzt wird.

Es bestand somit die Aufgabe, ein Verfahren zur Herstellung von Trimethylolverbindungen in Gegenwart von tertiären Aminen als Base bereitzustellen, welches eine Überführung der anfallenden Formiatsalze in eine verwertbare Form unter Rückführung des eingesetzten Amins erlaubt.

Überraschenderweise wurde nun ein Verfahren zur Herstellung von Trimethylolverbindungen und Ameisensäure durch Umsetzung von Formaldehyd und Aldehyden in Gegenwart einer Stickstoffbase gefunden, welches dadurch gekennzeichnet ist, dass nach der Umsetzung die Trimethylolverbindung aus dem Produktgemisch entfernt wird und anschließend die bei der Umsetzung entstehenden Formiatsalze durch Destillation in Gegenwart eines Hilfsstoffes in die freie Stickstoffbase und Ameisensäure aufgetrennt werden.

Durch das erfindungsgemäße Verfahren werden die dabei entstehenden Formiatsalze durch Destillation in Gegenwart eines Hilfsstoffes in Ameisensäure und die freie Stickstoffbase, welche vorteilhaft in das Verfahren zurückgeführt werden kann, aufgetrennt. Weiterhin ist durch die vorherige Entfernung der Trimethylolverbindung aus dem Produktgemisch sichergestellt, dass sich während der Auftrennung der Formiatsalze in Ameisensäure und die freie Stickstoffbase keine Trimethylol-Ameisensäureester bilden.

Ameisensäure ist ein wichtiges Produkt, welches beispielsweise in der Ledergerbung, zur Adjustierung von pH-Werten, in der Farbstoffherstellung und zur Herstellung von pharmazeutischen Produkten eingesetzt wird. Darüber hinaus wird Ameisensäure in der Koagulation von Kautschuk, als Zusatz zur Herstellung von Silofutter und als Promotor in Fermentationsprozessen verwendet.

Bei den im erfindungsgemäßen Verfahren eingesetzten Aldehyden handelt es sich vorzugsweise um solche der Formel (I) 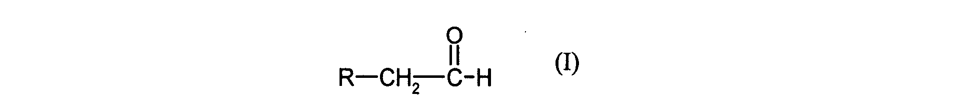 worin
- R: für Methylol, geradkettiges oder verzweigtes C₁-C₁₂-Alkyl, C₃-C₈-Cycloalkyl, C₆-C₁₀-Aryl oder C₇-C₂₂-Aralkyl steht, wobei die genannten Reste gegebenenfalls weitere, unter den Reaktionsbedingungen inerte Substituenten, wie Alkylgruppen oder Alkoxygruppen mit 1-3 C-Atomen, aufweisen können.

Steht R für einen Rest aus der Reihe geradkettiges oder verzweigtes C₁-C₁₂-Alkyl, so handelt es sich beispielsweise um Methyl, Ethyl, Propyl, Isopropyl, n-Butyl, sec.-Butyl, tert.-Butyl, Pentyl oder Hexyl. Steht R für einen Rest aus der Reihe C₃-C₈-Cycloalkyl, so handelt es sich beispielsweise um Cyclopropyl, Cyclobutyl, Cyclopentyl, Cyclohexyl, Cycloheptyl oder Cyclooctyl. Steht R für einen Rest aus der Reihe C₆-C₁₀-Aryl, so handelt es sich beispielsweise um Phenyl oder Naphthyl. Steht R für einen Rest aus der Reihe C₇-C₂₂-Aralkyl, so handelt es sich beispielsweise um Benzyl.

Im erfindungsgemäßen Verfahren werden insbesondere Aldehyde der Formel (I) eingesetzt, worin R für Methylol oder geradkettiges oder verzweigtes C₁-C₆-Alkyl steht. Besonders bevorzugt werden Aldehyde der Formel (I) eingesetzt, worin R für Methylol, Methyl, Ethyl, n-Propyl oder iso-Propyl und ganz besonders bevorzugt für Ethyl steht.

Der im erfindungsgemäßen Verfahren eingesetzte Formaldehyd kann gasförmig, in polymerer Form oder in Form einer wässrigen Lösung eingesetzt werden. Wird Formaldehyd im erfindungsgemäßen Verfahren in polymerer Form eingesetzt, so wird vorzugsweise Paraformaldehyd verwendet. Vorzugsweise wird Formaldehyd im erfindungsgemäßen Verfahren in Form einer wässrigen Lösung eingesetzt. Dabei handelt es sich vorzugsweise um eine 1 bis 55 gew.-%ige wässrige Lösung, bevorzugt um eine 5 bis 35 gew.-%ige Lösung, besonders bevorzugt um eine 10 bis 32 gew.-%ige Lösung.

Im erfindungsgemäßen Verfahren wird Formaldehyd vorzugsweise im Überschuss, bezogen auf den eingesetzten Aldehyd, eingesetzt. Vorzugsweise beträgt das Molverhältnis zwischen Aldehyd und Formaldehyd 1:3-10, besonders bevorzugt 1:3-5, ganz besonders bevorzugt 1:3-3,5.

Im erfindungsgemäßen Verfahren können als Stickstoffbasen solche eingesetzt werden, die als basische Katalysatoren für Aldolkondensationen bekannt sind und darüber hinaus eine Cannizzaro-Reaktion zwischen dem eingesetzten Aldehyd und Formaldehyd ermöglichen. Dabei handelt es sich beispielsweise um symmetrische Trialkylamine wie Trimethylamin, Triethylamin, Tri-n-propylamin, Triisopropylamin, Tri-n-butylamin, Triisobutylamin oder Tri-tert.-butylamin; unsymmetrische Trialkylamine wie beispielsweise Ethyldimethylamin, Isopropyl-dimethylamin, Diethylmethylamin, Dimethylpropylamin, Isobutyldimethylamin, Butyldimethylamin, tert.-Butyldimethylamin, Dimethylpentylamin, (2,2-Dimethylpropyl)-dimethylamin, Hexyldimethylamin oder Dibutylheptylamin; Diamine wie beispielsweise N,N,N',N'-Tetramethylbutan-1,3-diamin, Ethyldiiso-propyldiamin oder N,N,N',N'-Tetramethylethan-1,2-diamin; Allylamine wie beispielsweise Allyldimethylamin, Allyldiethylamin oder Triallylamin; Aminoalkohole wie beispielsweise 4-Dimethylaminoethanol, 2-Dimethylaminobutanol, 2-Diisopropyl-aminoethanol, Diethylaminomethanol, Diethylaminoethanol, 3-Dimethylaminopropan-1-ol oder Dimethylamino-2-methylpropan-1-ol; Alkoxy-substituierte Amine wie beispielsweise (2-Methoxyethyl)-dimethylamin, (3-Methoxypropyl)-dimethylamin oder Diethylmethoxymethylamin sowie Hydroxylamine wie beispielsweise N,N-Dimethylhydroxylamin.

Bevorzugt werden symmetrische Tri-n-alkylamine wie beispielsweise Trimethylamin, Triethylamin, Tri-n-propylamin oder Tri-n-butylamin eingesetzt, besonders bevorzugt Trimethylamin und Triethylamin.

Im erfindungsgemäßen Verfahren können auch Mischungen verschiedener Stickstoffbasen eingesetzt werden.

Im erfindungsgemäßen Verfahren wird die Stickstoffbase, bezogen auf 1 mol Aldehyd, vorzugsweise in einer Menge von 1 bis 10 mol, besonders bevorzugt von 1 bis 5 mol, ganz besonders bevorzugt von 1 bis 3 mol eingesetzt.

Im ersten Schritt des erfindungsgemäßen Verfahrens wird ein Aldehyd, vorzugsweise ein Aldehyd der Formel (I) mit Formaldehyd, vorzugsweise mit einer wässrigen Formaldehydlösung, in Gegenwart einer Stickstoffbase zu einer Trimethylol-verbindung und dem Formiatsalz der eingesetzten Stickstoffbase umgesetzt.

Die Umsetzung findet vorzugsweise bei Temperaturen von 10 bis 150°C, besonders bevorzugt bei 30 bis 130°C, ganz besonders bevorzugt bei 40 bis 100°C, statt.

Die Umsetzung kann bei Atmosphärendruck, vermindertem oder erhöhtem Druck durchgeführt werden. Wenn die gewählte Reaktionstemperatur den Siedepunkt der Komponenten der Reaktionsmischung übersteigt, kann die Umsetzung unter erhöhtem Druck durchgeführt werden. Vorzugsweise wird bei Atmosphärendruck gearbeitet.

Die Umsetzung kann sowohl batchweise, semibatcheise also auch kontinuierlich betrieben werden. Vorzugsweise wird sie kontinuierlich betrieben. Als Reaktionsapparate kommen alle dem Fachmann bekannten Reaktionsapparate, die zur Umsetzung von flüssigen Reaktanden geeignet sind, in Frage. Vorzugsweise wird die Umsetzung in Rührkesselreaktoren, Rührkesselkaskaden, Strömungsrohren oder Mehrkammerreaktoren durchgeführt.

Die Verweilzeit des Reaktionsgemisches im Reaktor kann beispielsweise 10 Minuten bis 50 Stunden betragen.

In einem zweiten Schritt des erfindungsgemäßen Verfahrens wird die entstandene Trimethylolverbindung aus dem Produktgemisch entfernt. Vorzugsweise wird die Trimethylolverbindung durch Extraktion aus dem Produktgemisch entfernt.

Wird die Trimethylolverbindung durch Extraktion aus dem Produktgemisch entfernt, so können als Extraktionsmittel Alkane, Cycloalkane, Alkohole, Ether, Aldehyde, Ketone oder Ester eingesetzt werden. Vorzugsweise werden Hexan, Cyclohexan, Isopropylalkohol, Isobutylalkohol, 2-Ethylhexanol, 2-Ethyl-2-hexenol, Cyclo-hexanol, tert.-Butylmethylether, Butyraldehyd, Propionaldehyd, Methylethylketon, Methylisobutylketon, Ethylacetat oder Butylacetat eingesetzt. Besonders bevorzugt wird derjenige Aldehyd als Extraktionsmittel eingesetzt, der im erfindungsgemäßen Verfahren zur Trimethylolverbindung umgesetzt wird. Ganz besonders bevorzugt wird Butyraldehyd als Extraktionsmittel eingesetzt.

Die Extraktion kann in jedem dem Fachmann bekannten Extraktionsapparat kontinuierlich oder diskontinuierlich durchgeführt werden. Bevorzugt wird die Extraktion kontinuierlich durchgeführt, vorzugsweise in einer Mixer-Settler-Apparatur, Siebboden- oder Packungskolonne, pulsierenden Siebboden oder Packungskolonne, Karrkolonne, Kühnikolonne, Spraykolonne oder in einem Zentrifugalextraktor.

Das bei der Extraktion erhaltene Gemisch aus Extraktionsmittel und Trimethylolverbindung wird bevorzugt destillativ aufgetrennt, besonders bevorzugt durch Rektifikation.

Nach der Entfernung der Trimethylolverbindung wird im erfindungsgemäßen Verfahren die verbleibende Formiatlösung mit einem Hilfsstoff versetzt, welcher die Aufgabe hat, die Ameisensäure zu binden, wodurch die Stickstoffbase freigesetzt wird.

Die Auftrennung von Formiatsalzen einer Stickstoffbase in Ameisensäure und Stickstoffbase unter Einsatz von Basen ist beispielsweise in EP 181 078 A beschrieben.

Im erfindungsgemäßen Verfahren werden als Hilfsstoffe vorzugsweise Substanzen eingesetzt, welche eine geringere Basizität aufweisen als die eingesetzte Stickstoffbase und in der Lage sind, mit Ameisensäure Addukte zu bilden, die bei einer höheren Temperatur als dem Siedepunkt der Stickstoffbase thermisch zersetzbar sind und die eine geringe Flüchtigkeit besitzen. Als Hilfsstoffe werden vorzugsweise Verbindungen eingesetzt, die Stickstoff enthalten und einen pK_{b} von 10 bis 3 besitzen.

Vorzugsweise werden im erfindungsgemäßen Verfahren als Hilfsstoffe cyclische Stickstoffverbindungen aus der Reihe Imidazole, Chinoline, Pyridine, Pyrimidine, Pyrrole, Pyrazole, Isochinoline, Pyrazine, Pyridazine, Piperidine, Pyrrolidine und Morpholine eingesetzt, wobei diese Verbindungen gegebenenfalls einen oder mehrere Substituenten aus der Reihe C₁-C₆-Alkyl, vorzugsweise Methyl, Formyl oder Phenyl tragen können wie beispielsweise 2-, 3- und 4-Methylpyridin, N-Methylmorpholin, N-Formylmorpholin oder N-Phenylmorpholin. Bevorzugte cyclische Stickstoffverbindungen sind N-Formylmorpholin, Dimorpholinethan, Chinolin und Imidazole der Formel (II) 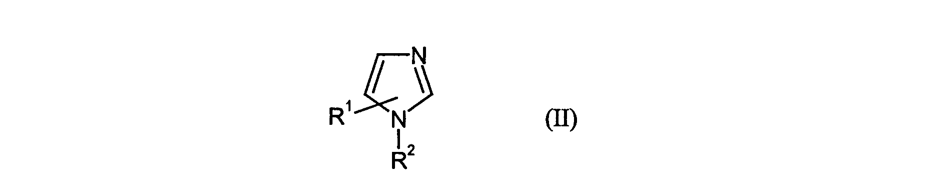 worin
- R¹ und R²: unabhängig voneinander für Wasserstoff oder für geradkettiges oder verzweigtes C₁-C₂₄-Alkyl stehen, vorzugsweise stehen R¹ und R² unabhängig voneinander für geradkettiges oder verzweigtes C₁-C₁₀-Alkyl wie beispielsweise Methyl, Ethyl, Propyl, Isopropyl, n-Butyl, sec.-Butyl, tert.-Butyl, n-Pentyl, n-Hexyl oder n-Heptyl.

In einer weiteren bevorzugten Ausführungsform werden Hilfsstoffe Amide der Formel (III) eingesetzt, 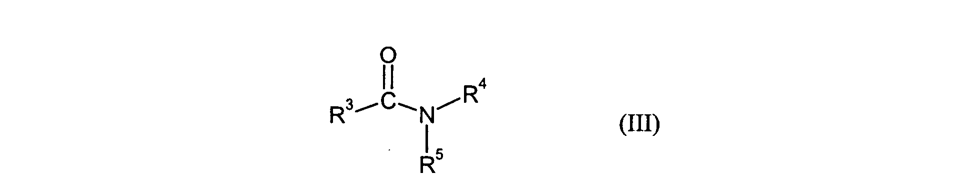 worin
- R³ und R⁴: unabhängig voneinander für geradkettiges oder verzweigtes C₁-C₂₄-Alkyl, vorzugsweise für geradkettiges oder verzweigtes C₁-C₁₀-Alkyl wie beispielsweise Methyl, Ethyl, Propyl, Isopropyl, n-Butyl, sec.-Butyl, tert.-Butyl, n-Pentyl, n-Hexyl oder n-Heptyl, für C₆-C₁₀-Aryl wie beispielsweise Phenyl oder Naphthyl oder für C₇-C₂₂-Aralkyl wie beispielsweise Benzyl stehen und
- R⁵: für Wasserstoff steht oder die Bedeutung von R³ und R⁴ hat.

In einer weiteren bevorzugten Ausführungsform werden als Hilfsstoffe cyclische Amide der Formel (IV) eingesetzt, 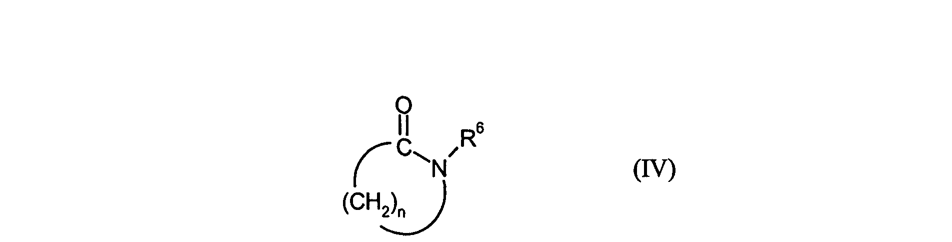 worin
- R⁶: für geradkettiges oder verzweigtes C₁-C₂₀-Alkyl wie beispielsweise Methyl, Ethyl, Propyl, Isopropyl, n-Butyl, sec.-Butyl, tert.-Butyl, n-Pentyl, Isopentyl, Hexyl, Isohexyl, Nonyl, n-Decyl, Dodecyl, Tridecyl, Tetradecyl, Hexadecyl, Octadecyl oder 2-Methylbutyl oder für einen C₂-C₂₀-Alkenylrest, wie beispielsweise Vinyl, Allyl oder Buten-2-yl steht und
- n: für eine Zahl von 3 bis 6 steht

Vorzugsweise werden als Hilfsstoffe cyclische Amide der Formel (IV) eingesetzt, worin n für 3 steht, besonders bevorzugt cyclische Amide der Formel (IV), worin n für 3 und R⁶ für Methyl oder Ethyl steht.

Weiterhin werden als Hilfsstoffe vorzugsweise cyclische Amide der Formel (IV) eingesetzt, worin n für 4 steht, besonders bevorzugt cyclische Amide der Formel (IV), worin n für 4 und R⁶ für Methyl oder Ethyl steht.

Ganz besonders bevorzugte Verbindungen, welche als Hilfsstoffe im erfindungsgemäßen Verfahren eingesetzt werden, sind N-Methylpyrrolidon, 1,2-Dimorpholinethan, N-Formylmorpholin, N-Methylacetamid, N,N-Dimethylacetamid, N-Ethylacetamid, N-Butylimidazol und N,N-Diethylacetamid.

Die Hilfsstoffe werden im erfindungsgemäßen Verfahren bezogen auf die Ameisensäure vorzugsweise im Überschuss eingesetzt. Besonders bevorzugt werden pro mol Ameisensäure 1,1 - 5 mol Hilfsstoff eingesetzt.

Vorzugsweise wird nach der destillativen Auftrennung des im erfindungsgemäßen Verfahren entstandenen Formiatsalzes der eingesetzten Stickstoffbase in Gegenwart eines Hilfsstoffes in die freie Stickstoffbase und Ameisensäure die Stickstoffbase in den Reaktionsprozess zurückgeführt.

Die Destillation erfolgt vorzugsweise bei Temperaturen von 10 bis 300°C, besonders bevorzugt bei 50 bis 250°C und vorzugsweise bei einem Druck von 1 mbar bis 5 bar, besonders bevorzugt 1 mbar bis 1 bar.

Nach destillativer Abtrennung der Base, welche vorzugsweise in den Reaktionsprozess zurückgeführt wird, wird die Ameisensäure vorzugsweise thermisch vom Hilfsstoff getrennt. In einer besonders bevorzugten Ausführungsform wird diese Trennung durch Extraktionsrektifikation erreicht, da hierbei eine sehr reine Ameisensäure gewonnen werden kann.

Die Abtrennung der Ameisensäure vom Hilfsstoff wird vorzugsweise bei einer Temperatur von 100 bis 300°C und einem Druck von 1 bis 200 mbar durchgeführt.

Der Sumpf aus Hilfsstoff und eventuell geringen Anteilen an Ameisensäure wird vorzugsweise in den Prozess zurückgeführt.

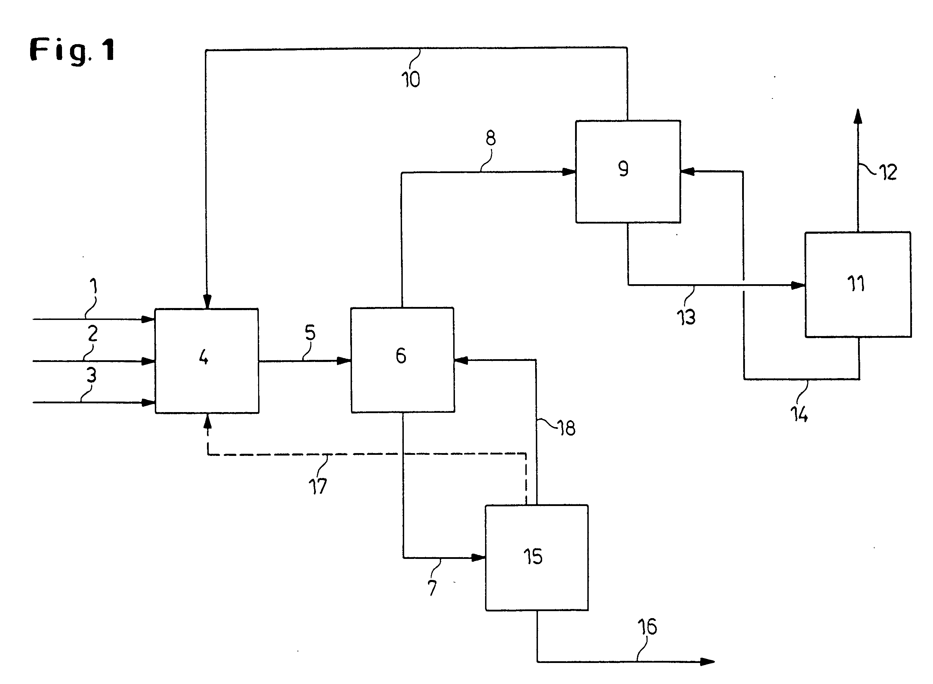

In **Fig. 1** ist eine bevorzugte Ausführungsform des erfindungsgemäßen Verfahrens dargestellt:

Der im erfindungsgemäßen Verfahren eingesetzte Aldehyd wird über den Strom **1** zusammen mit Formaldehyd und Stickstoffbase, welche größtenteils aus der Rückführung **10** stammt und zum geringen Teil über den Strom **3** frisch zugesetzt wird, einer Reaktionsstufe **4** zugeführt. In diese Reaktionsstufe wird der Aldehyd vorzugsweise bei Temperaturen von 10 bis 150°C umgesetzt. Die entstandene Reaktionsmischung wird als Strom **5** einer Trennstufe **6** zugeführt, in der die entstandene Trimethylolverbindung, vorzugsweise durch Extraktion, aus dem Produktgemisch entfernt wird. Zuvor können gegebenenfalls Leichtsieder und/oder ein Teil des Wassers aus dem Produktstrom destillativ entfernt werden, was in Fig. 1 nicht eingezeichnet ist. Bei den genannten Leichtsiedern handelt es sich beispielsweise um nicht vollständig umgesetzten Aldehyd, Formaldehyd oder Stickstoffbase, aber auch um Nebenprodukte wie beispielsweise Acroleine, im Fall der Trimethylolpropan-herstellung um α-Ethylacrolein. Werden Leichtsieder aus dem Produktstrom destillativ entfernt, so kann das Destillat gegebenenfalls ganz oder zum Teil in die Reaktionsstufe **4** zurückgeführt werden.

Wurde in einer bevorzugten Ausführungsform die Trimethylolverbindung durch Extraktion aus dem Produktgemisch entfernt, so wird der Extrakt in einer nachfolgenden Verfahrensstufe **15** in die Trimethylolverbindung (Strom **16**) und das Extraktionsmittel aufgetrennt, wobei die Auftrennung vorzugsweise durch Rektifikation erfolgt. Das eingesetzte Extraktionsmittel wird vorzugsweise als Strom **18** in die Extraktionsstufe **6** zurückgeführt oder, wenn der im erfindungsgemäßen Verfahren eingesetzte Aldehyd als Extraktionsmittel verwendet wird, ganz oder teilweise als Strom **17** in die Reaktionsstufe **4** zurückgeführt. Wird der im erfindungsgemäßen Verfahren eingesetzte Aldehyd als Extraktionsmittel eingesetzt, so wird in einer weiteren bevorzugten Ausführungsform der frisch zugeführte Aldehyd des Stromes **1** zuerst zum Extraktionsschritt **6** zugegeben.

Die bei der Entfernung der Trimethylolverbindung aus dem Produktgemisch erhaltene wässrige Raffinatphase **8**, die das Formiatsalz enthält, wird erfindungsgemäß durch eine Destillation in Gegenwart eines Hilfsstoffes in die freie Stickstoffbase und Ameisensäure überführt. Vorzugsweise wird dazu das Raffinat in einer Rektifikationskolonne **9** in Kontakt mit dem Hilfsstoff gebracht. Der Hilfsstoff wird vorzugsweise als Strom **14** in den oberen Teil der Kolonne eingespeist. In einer bevorzugten Ausführungsform wird die wässrige Raffinatphase **8** in den mittleren Teil einer Rektifikationskolonne **9** eingespeist, so dass der vorzugsweise in den oberen Teil der Kolonne eingespeiste Hilfsstoffstrom **14** der wässrigen Raffinatphase entgegengeleitet wird. In einer bevorzugten Ausführungsform wird die Rektifikationskolonne **9** bei Drücken von 100 - 1 000 mbar betrieben. Das in **9** erhaltene Destillat, welches die im Verfahren eingesetzte Stickstoffbase und gegebenenfalls restliches Wasser und Leichtsieder enthält, wird vorzugsweise direkt oder nach einer destillativen Reinigung, welche in Fig. 1 nicht eingezeichnet ist, als Strom **10** in die Reaktionsstufe **4** zurückgeführt.

Das Sumpfprodukt von **9** enthält im wesentlichen den im erfindungsgemäßen Verfahren eingesetzten Hilfsstoff und Ameisensäure. Der Sumpf wird vorzugsweise als Strom **13** in eine zweite Rektifikationskolonne **11** eingespeist, wobei die Einspeisung vorzugsweise in den mittleren Bereich der Kolonne erfolgt. Durch Rektifikation in der Rektifikationskolonne **11** wird das Sumpfprodukt aus **9** in die freie Ameisensäure als Kopfprodukt (Stoffstrom **12**) und Hilfsstoff als Sumpfprodukt (Stoffstrom **14**) getrennt. Die Rektifikation kann batchweise oder kontinuierlich durchgeführt werden. Bevorzugt wird die Rektifikation kontinuierlich durchgeführt. Die Rektifikation kann in allen dem Fachmann bekannten Rektifikationsapparaturen durchgeführt werden, bevorzugt werden Kolonnen mit Siebboden, Glocken-bodeneinbauten, Füllkörpern oder Packungskolonnen eingesetzt. Die reine Ameisensäure wird üblicherweise bei einem Druck von 50 bis 250 mbar und einer Temperatur von 20 bis 60°C erhalten. Der als Sumpfprodukt anfallende Hilfsstoff wird vorzugsweise in die Destillation **9** zurückgeführt.

### Beispiele

### Herstellung von Trimethylolpropan

### Beispiel 1

In einem 1 l Glasreaktor wurden 117,90 g destilliertes Wasser, 250,25 g (2,5 mol) 30 %-ige wässrige Formaldehyd-Lösung und 154,88 g (1,5 mol ) Triethylamin bei 25 °C vorgelegt. Anschließend wurden 36,46 g (0,5 mol) Butyraldehyd innerhalb von 45 min zudosiert und gleichzeitig die Reaktortemperatur linear auf 70°C angehoben. Nach Beendigung der Dosierung wurde 3 h unter Rückfluß nachgerührt. Man erhielt nach GC-Analytik Trimethylolpropan mit einer Ausbeute von 81,59 % der Theorie.

### Beispiel 2

In einem 1 l Glasreaktor wurden 117,90 g destilliertes Wasser, 250,25 g (2,5 mol) 30 %-ige wässrige Formaldehyd-Lösung und 77,44 g (0,75 mol ) Triethylamin bei 25 °C vorgelegt. Anschließend wurden 36,46 g (0,5 mol) Butyraldehyd innerhalb von 45 min zudosiert und gleichzeitig die Reaktortemperatur linear auf 70°C angehoben. Nach Beendigung der Dosierung wurde 3 h unter Rückfluß nachgerührt. Man erhielt nach GC-Analytik Trimethylolpropan mit einer Ausbeute von 82,77 % der Theorie.

### Beispiel 3

In einem 1 l Glasreaktor wurden 231,75 g destilliertes Wasser, 175,18 g (1,75 mol) 30 %-ige wässrige Formaldehyd-Lösung und 72,28 g (0,70 mol) Triethylamin bei 25 °C vorgelegt. Anschließend wurden 36,46 g (0,5 mol) Butyraldehyd innerhalb von 45 min zudosiert und gleichzeitig die Reaktortemperatur linear auf 70°C angehoben. Nach Beendigung der Dosierung wurde 4 h unter Rückfluß nachgerührt. Man erhielt nach GC-Analytik Trimethylolpropan mit einer Ausbeute von 76,34 % der Theorie.

### Extraktion von Trimethylolpropan

### Beispiel 4

100 g einer wäßrigen Trimethylolpropan-Lösung, welche 10 Gew.-% Trimethylolpropan und 15,4 Gew.-% Triethylammoniumformiat enthielt, wurden nacheinander dreimal mit 50 g n-Butyraldehyd extrahiert. Die vereinigten organischen Phasen (157,1 g) enthielten 7,7 g TMP und 0,1 g Triethylammoniumformiat.

### Rückgewinnung von Triethylamin

### Beispiel 5

Ein Gemisch aus N-Methylpyrrolidon (NMP), Wasser und Triethylammonium-formiat wurde in einer Destillationsapparatur, die aus einer 1,6 m hohen verspiegelten, mit 4 mm Geweberingen gefüllten Silbermantelkolonne und einer 1 l Blase bestand, destilliert. Zunächst wurden 623,8 g eines Gemisches bestehend aus 62,9 Gew.-% NMP, 22,3 Gew.-% Triethylammoniumformiat und 14,8 Gew.-% Wasser vorgelegt und bei Normaldruck destilliert. Bei einem Rücklaufverhältnis von 10 wurden 104,6 g Destillat mit einer Kopftemperatur von 76°C abgetrennt. Die Sumpftemperatur betrug 127 ― 136°C. Das Destillat bestand zu 88,3 Gew.-% aus Triethylamin und zu 11,7 Gew.-% aus Wasser. Die Wiedergewinnungsrate von Triethylamin betrug somit 92 %. Im Sumpf verblieben Ameisensäure und NMP.

### Beispiel 6

In der in Beispiel 5 beschrieben Apparatur wurden 560,7 g eines Gemischs bestehend aus 66,4 Gew.-% N-Butylimidazol, 20,8 Gew.-% Triethylammoniumformiat und 12,8 Gew.-% Wasser vorgelegt. Bei einem Rücklaufverhältnis von 10 wurden 79,2 g Destillat mit einer Kopftemperatur von 76°C abgetrennt. Das Destillat enthielt 82,3 Gew.-% Triethylamin und 17,7 Gew.-% Wasser. Die Wiedergewinnungsrate von Triethylamin betrug somit 85,8 %. Im Sumpf verblieben Ameisensäure und N-Butylimidazol.

### Isolierung von Ameisensäure

### Beispiel 7

In der in Beispiel 5 beschrieben Apparatur wurden 600 g eines Gemischs aus 16,7 Gew.-% Ameisensäure und 83,3 Gew.-% NMP vorgelegt. Bei 200 mbar und einem Rücklaufverhältnis von 10 wurden 52,6 g Destillat mit einer Kopftemperatur von 56 °C erhalten. Die Sumpftemperatur betrug 150 ― 152 °C. Das Destillat enthielt 98,6 Gew.-% Ameisensäure und 1,4 Gew.-% NMP.

## Patentansprüche

1. Verfahren zur Herstellung von Trimethylolverbindungen und Ameisensäure durch Umsetzung von Formaldehyd und Aldehyden in Gegenwart einer Stickstoffbase, **dadurch gekennzeichnet, dass** nach der Umsetzung die Trimethylolverbindung aus dem Produktgemisch enfernt wird und anschließend die bei der Umsetzung entstehenden Formiatsalze durch Destillation in Gegenwart eines Hilfsstoffes in die freie Stickstoffbase und Ameisensäure aufgetrennt werden.

2. Verfahren gemäß Anspruch 1, **dadurch gekennzeichnet, dass** es sich bei den eingesetzten Aldehyden um solche der Formel (I) handelt, 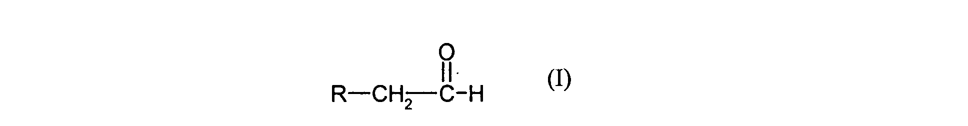 worin
R für Methylol, geradkettiges oder verzweigtes C₁-C₁₂-Alkyl, C₃-C₈-Cycloalkyl, C₆-C₁₀-Aryl oder C₇-C₂₂-Aralkyl steht, wobei die genannten Reste gegebenenfalls weitere, unter den Reaktions-bedingungen inerte Substituenten aufweisen.

3. Verfahren gemäß einem oder mehreren der vorangegangen Ansprüche 1 bis 2, **dadurch gekennzeichnet, dass** es sich bei dem eingesetzten Aldehyd um n-Butyraldehyd handelt.

4. Verfahren gemäß einem oder mehreren der vorangegangen Ansprüche 1 bis 3, **dadurch gekennzeichnet, dass** die Trimethylolverbindung durch Extraktion aus dem Produktgemisch entfernt wird.

5. Verfahren gemäß Anspruch 4, **dadurch gekennzeichnet, dass** zur Extraktion ein Extraktionsmittel aus der Reihe Alkane, Cycloalkane, Alkohole, Ether, Aldehyde, Ketone oder Ester eingesetzt wird.

6. Verfahren gemäß Anspruch 4 und 5, **dadurch gekennzeichnet, dass** als Extraktionsmittel derjenige Aldehyd eingesetzt wird, der gemäß Anspruch 1 mit Formaldehyd zur Trimethylolverbindung umgesetzt wird.

7. Verfahren gemäß einem oder mehreren der vorangegangen Ansprüche 1 bis 6, **dadurch gekennzeichnet, dass** es sich bei dem Hilfsstoff um Verbindungen handelt, welche eine geringere Basizität als die eingesetzte Stickstoffbase aufweisen und welche mit Ameisensäure Addukte bilden, die bei einer höheren Temperatur als dem Siedepunkt der eingesetzten Stickstoffbase thermisch zersetzbar sind.

8. Verfahren gemäß einem oder mehreren der vorangegangen Ansprüche 1 bis 7, **dadurch gekennzeichnet, dass** es sich bei dem Hilfsstoff um eine stickstoffhaltige Verbindung handelt, die einen pK_{b} von 10-3 aufweist.

9. Verfahren gemäß einem oder mehreren der vorangegangen Ansprüche 1 bis 8, **dadurch gekennzeichnet, dass** der Hilfsstoff ausgewählt ist aus
i) cyclische Stickstoffbasen aus der Reihe Imidazole, Chinoline, Pyridine, Pyrimidine, Pyrrole, Pyrazole, Isochinoline, Pyrazine, Pyridazine, Piperidine, Pyrrolidine und Morpholine,
ii) Amide der Formel (III), 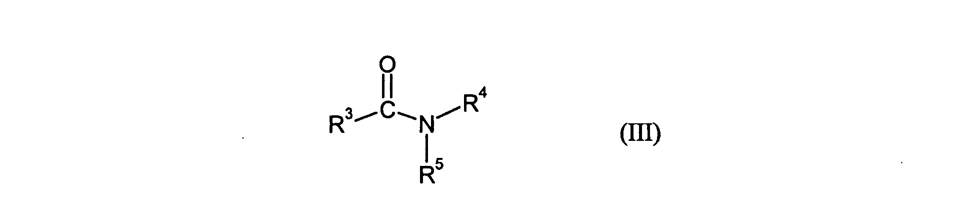 worin
R³ und R⁴ unabhängig voneinander für geradkettiges oder verzweigtes C₁-C₂₄-Alkyl, für C₆-C₁₀-Aryl oder für C₇-C₂₂-Aralkyl stehen und
R⁵ für Wasserstoff steht oder die Bedeutung von R³ und R⁴ hat,
iii) cyclische Amide der Formel (IV), 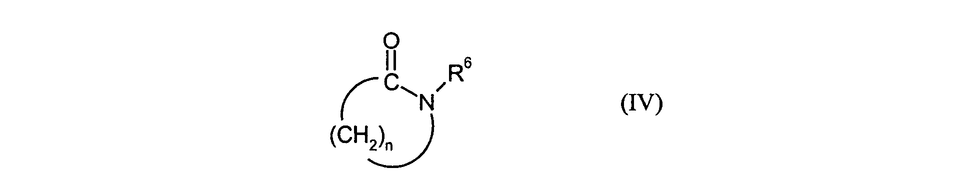
worin
R⁶ für geradkettiges oder verzweigtes C₁-C₂₀-Alkyl oder für einen C₂-C₂₀-Alkenylrest steht und
n für eine Zahl von 3 bis 6 steht.

10. Verfahren gemäß einem oder mehreren der vorangegangen Ansprüche 1 bis 9, **dadurch gekennzeichnet, dass** es sich bei dem Hilfsstoff um N-Methylpyrrolidon handelt.

11. Verfahren gemäß einem oder mehreren der vorangegangenen Ansprüche 1 bis 10, **dadurch gekennzeichnet, dass** die Destillation in Gegenwart eines Hilfsstoffes so durchgeführt wird, dass ein Kopfstrom anfällt, welcher die freie Stickstoffbase enthält und ein Sumpfprodukt anfällt, welches den Hilfsstoff und Ameisensäure enthält.

12. Verfahren gemäß Anspruche 11, **dadurch gekennzeichnet, dass** das Sumpfprodukt in einer anschließenden Destillation in den Hilfsstoff und die Ameisensäure aufgetrennt wird, wobei der Hilfsstoff in die Destillation zur Auftrennung des Formiatsalzes zurückgeführt wird.
